**Europäisches Patentamt**

**European Patent Office**

**Office européen des brevets**

(19)

(11) Numéro de publication: **0 329 521**
**B1**

(12)

# FASCICULE DE BREVET EUROPEEN

(45) Date de publication du fascicule du brevet:
**23.05.90**

(51) Int. Cl.⁵: **C07C 319/04**, C07C 321/04

(21) Numéro de dépôt: **89400329.2**

(22) Date de dépôt: **06.02.89**

(54) Perfectionnement à la synthèse de mercaptans tertiaires à partir d'homopolymères de l'isobutylène.

(30) Priorité: **17.02.88 FR 8801880**

(43) Date de publication de la demande:
**23.08.89 Bulletin 89/34**

(45) Mention de la délivrance du brevet:
**23.05.90 Bulletin 90/21**

(84) Etats contractants désignés:
**AT BE CH DE ES FR GB GR IT LI LU NL SE**

(56) Documents cités:
**FR-A- 2 531 426**
**US-A- 4 582 939**

(73) Titulaire: **SOCIETE NATIONALE ELF AQUITAINE (PRODUCTION), Tour Elf 2, Place de la Coupole La Défense 6, F-92400 Courbevoie(FR)**

(72) Inventeur: **Arretz, Emmanuel, 2 Rue de Cagnes, F-64000 Pau(FR)**

(74) Mandataire: **Leboulenger, Jean et al, ATOCHEM Département Propriété Industrielle, F-92091 Paris la Défense 10 Cédex 42(FR)**

## Description

La présente invention concerne le domaine des mercaptans et a plus particulièrement pour objet la fabrication de mercaptans tertiaires à partir d'homopolymères de l'isobutylène.

Il est connu que la formation de mercaptans tertiaires par réaction de l'hydrogène sulfuré avec des homopolymères de l'isobutylène est généralement accompagnée de produits secondaires, plus particulièrement de produits de décomposition de l'homopolymère d'isobutylène donnant à la fois des oléfines inférieures et les mercaptans correspondants. A cet égard on peut citer les brevets US 2 101 096, 2 426 646, 2 435 545 et 3 166 498 où, dans le cas du triisobutylène comme oléfine de départ, on observe la formation de tertiobutylmercaptan.

Bien qu'à un degré moindre, il en est de même du procédé décrit dans le brevet FR 2 531 426 qui concerne la synthèse de mercaptans en présence d'un catalyseur constitué par une résine échangeuse de cations et préconise de régler la température entre les limites rigoureuses de 45 et 75°C ou mieux entre 50 et 70°C. Ce procédé donne d'excellents résultats et est particulièrement performant pour l'obtention du tertiobutylmercaptan, du tertiononylmercaptan et du tertiododécylmercaptan à partir respectivement de l'isobutylène, du propylène trimère et du propylène tétramère. Cependant, dans le cas des homopolymères de l'isobutylène tels que, par exemple, le diisobutylène ou le triisobutylène, on observe encore une formation non négligeable de sous-produits légers dont la présence dans les produits de réaction nécessite des purifications par distillation et entraîne une perte finale de matière active par rapport à la production du mercaptan tertiaire recherchée.

Il a maintenant été trouvé que, dans le cas des homopolymères de l'isobutylène, on obtient de bien meilleurs résultats en effectuant la réaction à une température inférieure à 45°C. Des résultats surprenants ont été notamment obtenus à des températures aussi basses que 5-10°C pour lesquelles la sélectivité en mercaptan correspondant à l'homopolymère de départ pratiquement totale, sans affecter de manière sensible la vitesse de réaction, donc la productivité du réacteur.

Le procédé selon l'invention pour la préparation de mercaptans tertiaires par réaction d'hydrogène sulfuré avec un homopolymère de l'isobutylène est donc caractérisé en ce que l'on effectue la réaction à une température inférieure à 45°C, de préférence entre 0 et 35°C, en présence d'un catalyseur constitué par une résine échangeuse de cations sèche.

Bien qu'il soit possible d'opérer à toute pression comprise entre 1 et 50 bars, il et préférable industriellement de travailler à une pression allant de 5 à 16 bars. Les basses températures mises en oeuvre selon l'invention favorisent particulièrement la dissolution de l'hydrogène sulfuré dans les homopolymères d'isobutylène et permettent d'opérer à des pressions modérées, notamment inférieures à 10 bars.

Comme dans les procédés connus, il convient d'employer un certain excès d'hydrogène sulfuré, les rapports molaires pratiques $H_2S$/homopolymère d'isobutylène étant compris entre 1,2 et 10 et, de préférence, entre 1,5 et 5.

Le procédé selon l'invention peut être appliqué à des homopolymères d'isobutylène ayant jusqu'à 20 atomes de carbone. Il présente cependant un intérêt tout particulier pour la fabrication des mercaptans dérivés de diisobutylène, de triisobutylène et de tétraisobutylène.

Comme catalyseur à utiliser selon l'invention conviennent tous les différents polymères et copolymères à fonctions acides, connus dans l'art comme échangeurs de cations. En particulier, on peut employer des résines à base de polystyrène sulfoné réticulées, en particulier avec du divinylbenzène, des résines acryliques ou phénylacryliques à groupes carboxyliques libres, des résines du type phénol-formaldéhyde dérivées des acides phénol-sulfoniques, des échangeurs ligno-sulfoniques, etc... Des résines de ce genre se trouvent dans le commerce sous différentes dénominations, en particulier Allassion, Cecacit, Wofatites, Levatites, Imac, Ionac, Amberlites, Liquorex, Zeorex, Zeocarb, Dowex, etc... Conviennent tout particulièrement les copolymères sulfonés du styrène avec le divinyl benzène, par exemple ceux que l'on trouve dans le commerce sous les dénominations Amberlyst, Lewatit ou Dowex ; d'autre part, peuvent être employés avantageusement les copolymères de tétrafluoroéthylène avec un acide perfluorosulfonique (en particulier l'acide perfluoro-3,6-dioxa-4-méthyl-7-octène sulfonique) connus sous la marque Nafion. Quelle que soit la résine employée comme catalyseur, il faut veiller à ce qu'elle ne contienne pas plus de 0,5 % d'eau déterminable après 6 heures de séchage à 80°C et soit de préférence aussi sèche que possible (avantageusement moins de 0,2 % d'eau).

Le procédé selon l'invention peut être mis en oeuvre en discontinu ou en continu suivant toute méthode connue en soi. On préfère cependant opérer en continu, dans un réacteur agité ou dans un réacteur tubulaire, chargé de catalyseur et alimenté de manière continue en hydrogène sulfuré et en homopolymère d'isobutylène. Dans certains cas, il peut être avantageux de recycler au réacteur l'homopolymère non transformé.

Les exemples suivants illustrent l'invention, sans la limiter.

## EXEMPLE 1 : SYNTHESE DU TERTIO-OCTYLMERCAPTAN

### a) Selon la technique antérieure

Dans un réacteur tubulaire d'une capacité d'environ 420 ml (longueur : 135 cm ; diamètre intérieur : 20 mm) on place 200 ml de résine sulfonée Amberlyst 15 sèche. Sous une pression de 10 bars on introduit en tête du réacteur, en continu, du diisobutylène liquide à raison de 112 g/h (soit 1 mole/h) et 136 g/h de $H_2S$ gazeux (soit 4 moles de $H_2S$ pour 1 de diisobutylène).

Les réactifs sont mélangés intimement avant leur passage dans le réacteur où le mélange réactionnel est maintenu à la température de 45°C. Le liquide s'écoulant en continu du réacteur est recueilli et l'hydrogène sulfuré restant est dégazé. Des analyses sont effectuées à la fois sur les bruts de réaction liquides et sur les effluents gazeux de réaction dans le but de faire le bilan complet de la réaction.

La formation de sous-produits étant importante (environ 15 %), un second essai a été effectué à la même température de 45°C, mais cette fois en opérant avec des débits de réactifs 4 fois supérieurs dans le but d'améliorer la sélectivité en tertio-octylmercaptan en réduisant le temps en contact des produits sur le catalyseur. On n'obtient pratiquement pas de différence par rapport au premier essai.

Les résultats de ces deux essais sont présentés dans le tableau suivant :

| DIB (mole/h) | Conversion DIB | Rendement TOM | Rendement TBM | Autres sous-produits |
|---|---|---|---|---|
| 1 | 98 | 81,2 | 4,2 | ≃11 |
| 4 | 97,8 | 80,9 | 3,5 | ≃12 |

DIB: diisobutylène
TOM: tertio-octylmercaptan
TBM: tertio-butylmercaptan

### b) Selon l'invention

En opérant comme précédemment avec un débit de diisobutylène de 448 g/h et un débit de $H_2S$ de 544 g/h, on a réalisé quatre opérations à des températures inférieures à 45°C, à savoir 5, 10, 20 et 30°C.

| Temperature (°C) | Conversion DIB | Rendement TOM | Rendement TBM | Autres sous-produits |
|---|---|---|---|---|
| 30 | 97,6 | 91 | 0,2 | 5 |
| 20 | 97,3 | 92,5 | 0 | 4 |
| 10 | 97,1 | 93 | 0 | 3,6 |
| 5 | 96,5 | 94,2 | 0 | 2,5 |

On constate qu'à ces températures, tout en conservant un taux de conversion du diisobutylène du même ordre qu'à 45°C, on améliore nettement le rendement en tertio-octylmercaptan désiré, on élimine pratiquement la formation de tertio-butylmercaptan et diminue très nettement celle des autres sous-produits.

## EXEMPLE 2 : SYNTHESE DU TERTIO-DODECYLMERCAPTAN

Dans le même réacteur qu'à l'exemple 1, chargé de 200 ml de résine Amberlyst 15, on a effectué une série d'essais en introduisant en continu sous une pression de 10 bars, un mélange constitué de triisobutylène liquide à raison de 121 g/h (soit 0,72 mole/h) et de $H_2S$ à raison de 122,4 g/h (soit 3,6 moles/h). Pour chaque essai on a fait varier la température de réaction dans un intervalle compris entre 45°C et 10°C.

Le tableau suivant rassemble les compositions pondérales des produits identifiés analytiquement dans les différents effluents de réaction.

| Temperature (°C) | TIB (%) | TDM (%) | TOM (%) | TBM (%) |
|---|---|---|---|---|
| 45 | 43,6 | 20,4 | 18,1 | 8,2 |
| 30 | 49,6 | 30,5 | 9,1 | 3,0 |
| 20 | 50,4 | 41,3 | 0,8 | 0,15 |
| 10 | 50,7 | 49,2 | 0,1 | 0,05 |

TIB : triisobutylène
TDM : tertio-dodécylmercaptan
TOM : tertio-octylmercaptan
TBM : tertio-butylmercaptan

Par rapport au TIB effectivement consommé, le rendement en TDM à 10 et 20°C est de l'ordre de 85 %. Puisque la formation de TOM et de TBM à ces températures est très faible, on peut améliorer la productivité en recyclant le TIB non consommé, sans nuire à la qualité du TDM désiré.

## Revendications

1. Procédé de préparation de mercaptans tertiaires à partir d'homopolymères de l'isobutylène et d'hydrogène sulfuré par catalyse hétérogène, caractérisé en ce que l'on effectue la réaction à une température inférieure à 45°C en présence d'un catalyseur constitué par une résine échangeuse de cations sèche.

2. Procédé selon la revendication 1, dans lequel on opère à une température comprise entre 0 et 35°C.

3. Procédé selon la revendication 1 ou 2, dans lequel la résine échangeuse de cations est un copolymère sulfoné styrène-divinylbenzène ou un copolymère tétrafluoroéthylène-acide perfluorosulfonique.

4. Procédé selon l'une des revendications 1 à 3, dans lequel on opère sous une pression comprise entre 1 et 50 bars, de préférence entre 5 et 16 bars.

5. Procédé selon l'une des revendications 1 à 4, dans lequel le rapport molaire $H_2S$/homopolymère de l'isobutylène est compris entre 1,2 et 10, de préférence entre 1,5 et 5.

6. Procédé selon l'une des revendications 1 à 5, dans lequel l'homopolymère d'isobutylène est le diisobutylène, le triisobutylène ou le tétraisobutylène.

7. Procédé selon l'une des revendications 1 à 6, dans lequel on recycle l'homopolymère d'isobutylène non transformé.

## Claims

1. Process for the preparation of tertiary mercaptans from isobutylene homopolymers and from hydrogen sulphide by means of heterogeneous catalysis, characterized in that the reaction is carried out at a temperature below 45°C in the presence of a catalyst consisting of a dry cation exchange resin.

2. Process according to Claim 1, in which the operation is carried out at a temperature of between 0 and 35°C.

3. Process according to Claim 1 or 2, in which the cation exchange resin is a sulphonated styrene-divinylbenzene copolymer or a tetrafluoroethylene-perfluorosulphonic acid copolymer.

4. Process according to one of Claims 1 to 3, in which the operation is carried out at a pressure of between 1 and 50 bars, preferably between 5 and 16 bars.

5. Process according to one of Claims 1 to 4, in which the molar ratio $H_2S$/isobutylene homopolymer is between 1.2 and 10, preferably between 1.5 and 5.

6. Process according to one of Claims 1 to 5, in which the isobutylene homopolymer is diisobutylene, triisobutylene or tetraisobutylene.

7. Process according to one of Claims 1 to 6, in which the unconverted isobutylene homopolymer is recycled.

## Patentansprüche

1. Verfahren zur Herstellung tertiärer Mercaptane ausgehend von Homopolymeren des Isobutylens und Schwefelwasserstoff durch heterogene Katalyse, dadurch gekennzeichnet, daß man die Reaktion bei einer Temperatur unterhalb von 45°C in Gegenwart eines Katalysators bestehend aus einem trockenen Kationenaustauscherharz durchführt.

2. Verfahren nach Anspruch 1, bei dem man bei einer Temperatur zwischen 0 und 35°C arbeitet.

3. Verfahren nach Anspruch 1 oder 2, bei dem das Kationenaustauscherharz ein sulfoniertes Styrol-Divinylbenzol-Copolymer ist oder ein Tetrafluorethylen-Perfluorsulfonsäure-Copolymer ist.

4. Verfahren nach einem der Ansprüche 1 bis 3, bei dem man bei einem Druck zwischen 1 und 50 bar vorzugsweise zwischen 5 und 16 bar arbeitet.

5. Verfahren gemäß einem der Ansprüche 1 bis 4, bei dem das molare Verhältnis $H_2S$/Isobutylen-Homopolymer zwischen 1,2 und 10, vorzugsweise zwischen 1,5 und 5 beträgt.

6. Verfahren nach einem der Ansprüche 1 bis 5, bei dem das Isobutylen-Homopolymer Diisobutylen, Triisobutylen oder Tetraisobutylen ist.

7. Verfahren nach einem der Ansprüche 1 bis 6, bei dem man das nicht umgewandelte Isobutylen-Homopolymer zurückführt.